# EUROPEAN PATENT APPLICATION

(11) **EP 4 209 195 A1**
(43) Date of publication of application: **12.07.2023**
(21) Application number: 21883348.1
(22) Date of filing: 22.10.2021
(51) Int. Cl.: A61C 17/34, A61C 17/22, H02P 25/032, A46B 15/00, A46B 9/04, H02J 7/00

(54) **ELECTRIC TOOTHBRUSH HAVING CONDUCTIVE BRISTLES**

(30) Priority: 23.10.2020 KR 20200138612
(71) Applicant: Proxihealthcare Inc., Ulsan 44988 (KR)
(72) Inventor: KIM, Young Wook, Seoul 03741 (KR)
(74) Representative: AWA Sweden AB
(86) International application number: PCT/KR2021/014935
(87) International publication number: WO 2022/086281

(57) **Abstract**

This invention relates to an electric toothbrush including: a toothbrush head having a plurality of conductive bristles and at least one electrode supplied with a driving signal; a handle part connected to the toothbrush head and having a user-grippable shape; a battery disposed inside the handle part; and a signal supply located inside the handle part and configured to receive a voltage from the battery to generate an AC signal and a DC signal, and to generate the driving signal comprising a mixture of the AC signal and the DC signal to supply the electrode.

## Description

### TECHNICAL FIELD

The present invention relates to an electric toothbrush, and more particularly to an electric toothbrush for enhanced oral care effectiveness by providing microcurrents through conductive bristles.

### BACKGROUND ART

Dental plaque is a sticky and transparent film that adheres to the surface of teeth. The dental plaque is formed as numerous germs (bacteria) living in the mouth adhere to certain components in saliva. The dental plaque may be formed not only on and around the teeth, but also around prostheses, braces, and dentures.

When the dental plaque in the form of a very thin and transparent film is created, the bacteria in the plaque proliferate and also increase exponentially using the sugar supplied when food is consumed. The acidic substances produced by the bacteria in the plaque dissolve the lime in the teeth, causing tooth decay, and the toxins cause inflammation in the gums.

The dental plaque itself is difficult to see with the naked eye, and it mainly occurs in deep valleys of teeth, narrow gaps between teeth, and narrow gaps between teeth and gums. Because the plaque causes problems to teeth and surrounding tissues in such a small space, it is important to remove the plaque without missing every corner, but there is a problem in that it is difficult to effectively remove such plaque using only a conventional toothbrush.

### DISCLOSURE

### TECHNICAL PROBLEM

An object of the present invention to solve the above problems is to provide an electric toothbrush capable of effectively removing dental plaque.

Further, another object of the present invention is to provide an electric toothbrush capable of preventing the development of tooth decay and periodontal disease through the removal of dental plaque.

Furthermore, another object of the present invention is to provide an electric toothbrush capable of extending electrical energy from the electrodes through the conductive bristles by co-locating the electrodes and the conductive bristles in the toothbrush head, thereby amplifying the effectiveness of oral care.

### TECHNICAL SOLUTION

An electric toothbrush according to an embodiment of the present invention may include a toothbrush head having a plurality of conductive bristles and at least one electrode disposed thereon that is supplied with a driving signal, a handle part connected to the toothbrush head and having a shape that is grippable by a user, a battery disposed in an interior of the handle part, and a signal supply that is disposed in the handle part and that is configured to receive a voltage input from the battery to generate an AC signal and a DC signal, and to generate a driving signal that is a mixture of the AC signal and the DC signal to supply the electrode.

Further, the conductive bristles may comprise a synthetic resin and a conductive material.

Further, the conductive material may comprise graphene.

Further, the conductive bristles may be spaced apart from the electrodes.

Further, in use of the electric toothbrush, the electrodes and the conductive bristles may be electrically connected through saliva and toothpaste in the oral cavity such that the driving signal from the electrodes is transmitted to the conductive bristles.

Further, the electrode may have a plurality of first bristle holes formed therein, and at least a portion of the conductive bristles may be electrically connected to the electrode by being inserted into the first bristle holes.

Further, the electric toothbrush may comprise an additional electrode spaced apart from the electrode and disposed on the toothbrush head, wherein the additional electrode may have a plurality of second bristle holes formed into which at least another portion of the conductive bristles are inserted.

Further, the additional electrode may be supplied with the driving signal or set to a ground potential.

Further, the signal supply may include a DC-DC converter that is configured to receive a voltage from the battery and convert it to an output voltage, a signal generator that is configured to generate the AC signal using the output voltage of the DC-DC converter, a filter that is configured to perform a filtering operation on the AC signal generated by the signal generator, and a calibration part that is configured to mix the DC signal with the AC signal supplied through the filter to generate the driving signal.

Further, the signal supply may further comprise a voltage divider configured to distribute an output voltage of the DC-DC converter to generate the DC signal.

Further, it may be characterized in that the frequency of the driving signal is between 1 KHz and 1,000 MHz.

Further, it may be characterized in that the amplitude of the driving signal is between 0.1 mv and 3 volts.

Further, the voltage value of the DC signal may be characterized in that it is set above the amplitude of the AC signal.

### ADVANTAGEOUS EFFECTS

According to the present invention, as discussed above, an electric toothbrush capable of effectively removing dental plaque can be provided.

Further, according to the present invention, an electric toothbrush can be provided that can prevent the development of tooth decay and periodontal disease through the removal of tooth plaque.

Further, according to the present invention, by disposing an electrode and a conductive bristle together in a toothbrush head, electrical energy from the electrode can be extended through the conductive bristle, thereby providing an electric toothbrush that can improve oral care effectiveness.

### BRIEF DESCRIPTION OF THE DRAWINGS

FIG. 1 is a view illustrating an electric toothbrush according to an embodiment of the present invention.
FIGS. 2A and 2B are views to illustrate the biofilm removal effect of a driving signal generated by mixing an AC signal with a DC signal.
FIG. 3 is a diagram illustrating a signal supply according to an embodiment of the present invention.
FIGS. 4A through 4C are diagrams illustrating waveforms of signals according to embodiments of the present invention.
FIGS. 5A and 5B are views illustrating a toothbrush head according to embodiments of the present invention.
FIGS. 6A and 6B are views illustrating a toothbrush head according to another embodiment of the present invention.
FIGS. 7A and 7B are views illustrating a toothbrush head according to another embodiment of the present invention.
FIG. 8 is a view of an electric toothbrush according to an embodiment of the present invention in concrete form.
FIG. 9 is a view of the electric toothbrush shown in FIG. 8 in a disassembled state.
FIG. 10 is a view of a head part according to an embodiment of the present invention.
FIGS. 11A through 11C are views illustrating a head cover according to embodiments of the present invention.
FIGS. 12A and 12B are views illustrating the coupling relationship of a head cover, a connecting wire, and a connecting pin according to embodiments of the present invention.
FIG. 13A is an exploded view illustrating an internal configuration of a handle part according to an embodiment of the present invention, FIG. 13B is a view illustrating a combined state of the components shown in FIG. 13A, and FIG. 13C is a view illustrating a retaining pin and a connection terminal according to an embodiment of the present invention.
FIG. 14 is a view illustrating the coupling relationship of a battery cap, an inner case, and an outer case in accordance with embodiments of the present invention.
FIG. 15 is a partial cross-sectional view of an electric toothbrush when the head and handle parts are combined, according to an embodiment of the present invention.

### DETAILED DESCRIPTION OF EXEMPLARY EMBODIMENTS

Hereinafter, embodiments of the present invention will be described in detail with reference to the accompanying drawings. However, the present invention is not limited to the embodiments disclosed below and may be implemented in various different forms. Also, it should be understood that all modifications, equivalents, or replacements thereof are included within the subject matter and scope of the present invention.

In describing elements of the present invention, terms such as first, second, A, B, (a), and (b) may be used. These terms are only used to distinguish one element from other elements, and the nature, sequence, or order of that element is not limited by the term. Further, it should be understood in this specification that if an element is described as being "connected", "combined", or "coupled" to/with any other element, the element may be directly connected, combined, or coupled to/with the other element, but another element may also be connected, combined, or coupled between both elements. In the case of being "connected", "combined", or "coupled", it may be understood as being physically or electrically connected, combined, or coupled, but is also electrically "connected", "combined", or "coupled" " as needed.

Terms such as "∼ part", "∼er", "part", and "∼ module" used in this specification refer to a unit that processes at least one particular function or operation, and may be implemented with hardware, software, or a combination thereof. In addition, terms such as "comprise", "include", and "have" used in this specification denote the presence of a stated element unless the relevant context clearly indicates otherwise, and do not exclude the presence of or a possibility of addition of one or more other elements.

In this specification, the distinction between elements is only a distinction by a main function performed by each element. That is, two or more elements to be described below may be combined into one element, or one element may be divided into two or more elements according to subdivided functions. Also, each element to be described below may further perform some or all of functions performed by other elements in addition to the main function thereof, and a part of the main function of each element may be performed by other elements.

Referring now to the following drawings, which relate to embodiments of the present invention, an electric toothbrush having conductive bristles (hereinafter referred to as an electric toothbrush) according to embodiments of the present invention will be described.

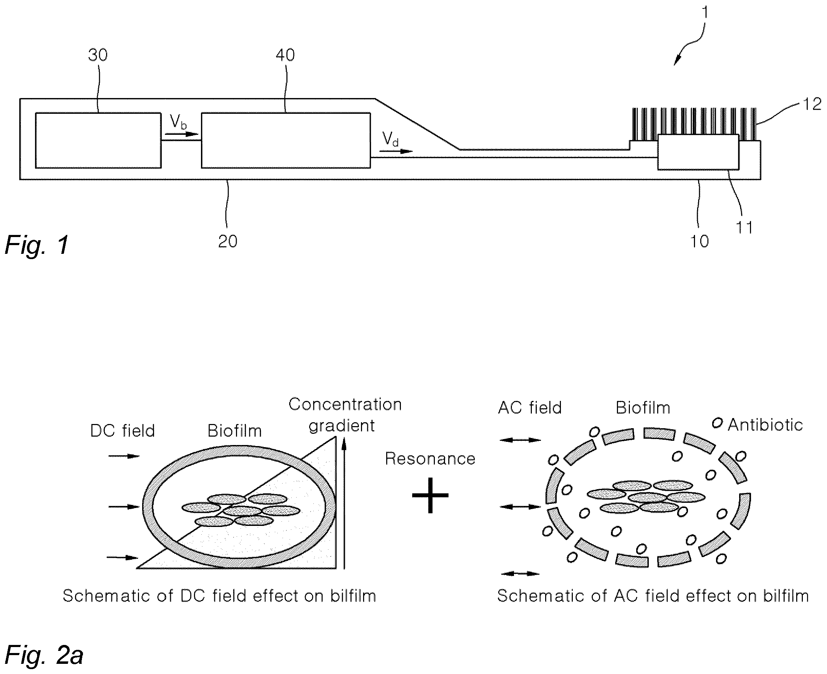

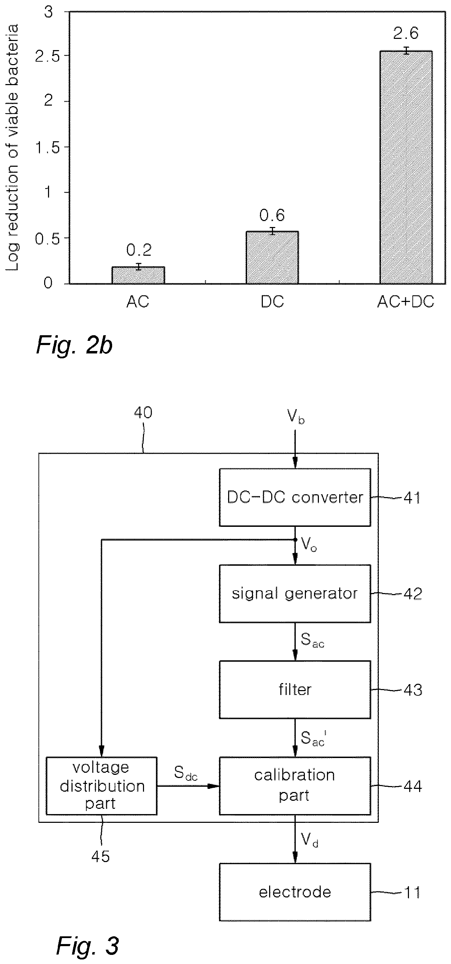

FIG. 1 is a view illustrating an electric toothbrush according to an embodiment of the present invention, and FIGS. 2A and 2B are views to illustrate the biofilm removal effect of a driving signal generated by mixing an AC signal and a DC signal.

Referring to FIG. 1, an electric toothbrush 1 according to an embodiment of the present invention may comprise a toothbrush head 10, a handle part 20, a battery 30, and a signal supply 40.

The toothbrush head 10 may comprise a plurality of conductive bristles 12 and at least one electrode 11.

Conductive bristles 12 may be disposed in plurality on the toothbrush head 10, and may be electrically conductive by containing a conductive material, unlike conventional ordinary bristles.

To this end, the conductive bristles 12 may be constructed including a synthetic resin and a conductive material, wherein the conductive material may include, for example, graphene.

Graphene has high electron mobility and electrical conductivity, and is also highly elastic, meaning it does not lose its electrical properties when stretched or bent, making it an ideal material for use as a toothbrush head. Graphene also has excellent strength, which contributes to the longevity of toothbrush bristles, and its transparent nature does not affect the color of the bristles.

Electrodes 11 may be disposed at least one on the toothbrush head 10, and may be electrically connected to a signal supply 40 disposed on the handle part 20 to be supplied with a driving signal Vd.

The electrodes 11 may form an electric field based on the electrical energy of the driving signal Vd. Such an electric field may weaken the structure of the dental plaque, so that the user can effectively remove the dental plaque in the oral cavity using the electric toothbrush 1.

Furthermore, while FIG. 1 illustrates the electrode 11 protruding from the toothbrush head 10, it is not limited to this. For example, a separate groove or hole may be formed in the toothbrush head 10, and the electrode 11 may be inserted inside said groove or hole. In this case, the electrode 11 may have a shape that does not protrude outwardly.

For example, the electrode 11 may be formed from a material such as, but not limited to, brass, aluminum, a conductive polymer, a conductive silicon, stainless steel, or the like; however, any material that is conductive may be used as the electrode material.

A handle part 20 is connected to the toothbrush head 10 and may have a shape that is grippable by a user. A separate button or switch (not shown) for operating the electric toothbrush 1 may be arranged on such handle part 20.

Additionally, a battery 30 and a signal supply 40 may be located inside the handle part 20.

A battery 30 may be housed within the handle part 20 and may provide a battery voltage Vb to the signal supply 40.

For example, the battery 30 may be set as a primary battery or a secondary battery.

If the battery 30 is a primary cell, the user may periodically replace the battery 30, and if the battery 30 is a secondary cell, charging may be accomplished through various charging methods.

For example, the battery 30 may be charged via a wireless charging method or a wired charging method while located within the handle part 20, and may also be detached from the handle part 20 and charged via a separate charging device.

The signal supply 40 is located inside the handle part 20, and can generate a driving signal Vd using a battery voltage Vb supplied from the battery 30.

In particular, the signal supply 40 may mix an alternating current (AC) signal and a direct current (DC) signal to generate the driving signal Vd.

Accordingly, the driving signal Vd includes both an AC component and a DC component, and a synergistic effect and resonance may occur due to the simultaneous application of the AC component and the DC component, thereby enhancing the removal effect of the biofilm that causes dental plaque.

Referring to FIG. 2A, an electric field with a DC component can increase the structural stress of the biofilm by inducing an imbalance in the local charge distribution, and an electric field with an AC component can increase the permeability of the outer protective material through the generation of specific vibrations.

The synergistic effect of these AC and DC components can be seen in FIG. 2B, which shows that the biofilm removal effect of the electric field with the AC component and the electric field with the DC voltage component is superior when the electric field with the AC component and the electric field with the DC voltage component are superimposed and applied simultaneously, compared to the biofilm removal effect of the electric field with each component alone.

Due to the driving signal Vd supplied by the signal supply 40 by an embodiment of the present invention, an electric field with a DC component and an electric field with an AC component can be simultaneously provided through the electrode 11, thereby achieving the amplified removal effect with respect to the biofilm described above.

Furthermore, by setting the driving signal Vd in a form in which the alternating current voltage and the direct current voltage are superimposed as described above, the risk of electric shock to the body and the pain that can be caused to the body can be reduced compared to the case in which only the direct current voltage is applied.

Furthermore, in the electric toothbrush 1 according to an embodiment of the present invention, the electrical energy by the driving signal Vd can be extended by using the bristles 12 having electrical conductivity.

In other words, by using the conductive bristles 12, the electrical energy caused by the driving signal Vd can be transmitted more efficiently to the teeth, gums, and the like in the oral cavity, thereby improving the effectiveness of oral care.

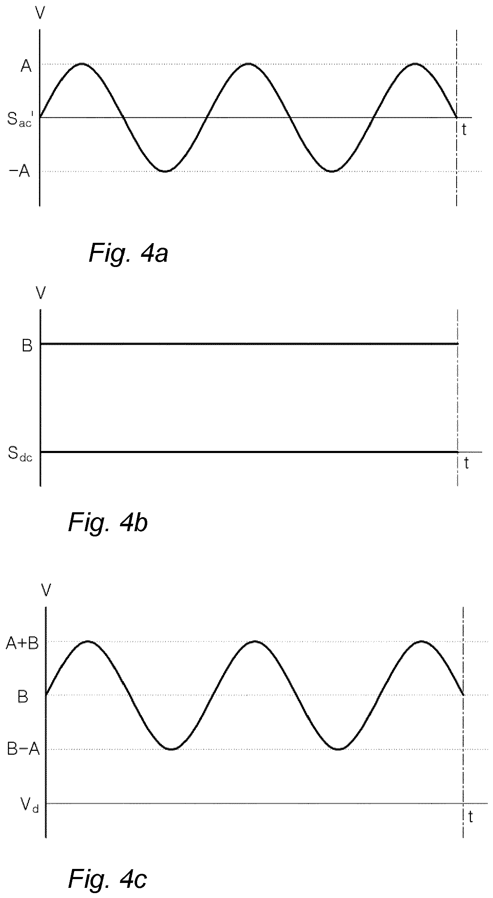

FIG. 3 is a diagram illustrating a signal supply by an embodiment of the present invention, and FIGS. 4A through 4C are diagrams illustrating waveforms of signals by embodiments of the present invention. In particular, FIG. 4A shows a filtered AC signal Sac', FIG. 4B shows a DC signal Sdc, and FIG. 4C shows a driving signal Vd generated by mixing the filtered AC signal Sac' and the DC signal Sdc.

Referring to FIG. 3, the signal supply 40 according to an embodiment of the present invention may include a DC-DC converter 41, a signal generator 42, a filter 43, and a calibration part 44, and may further include a voltage distribution part 45.

The DC-DC converter 41 may receive a battery voltage Vb from the battery 30 and convert the battery voltage Vb to an output voltage Vo at a predetermined level and output it.

The signal generator 42 operates based on a voltage supplied from the DC-DC converter 41, and can generate an AC signal Sac having a predetermined frequency using the output voltage Vo of the DC-DC converter 41.

The signal generator 42 may be implemented using any known configuration capable of generating an AC signal, such as an oscillator, function generator, or the like.

For example, the AC signal Sac may be set to a frequency of 1 KHz to 1,000 MHz. This is because if the AC signal Sac is set to a low frequency of less than 1 KHz, the removal effect of dental plaque is reduced, and if the AC signal Sac is set to an ultra-high frequency of more than 1,000 MHz, the removal effect of dental plaque is also reduced. On the other hand, the frequency of the AC signal Sac can be set to a frequency of 5 MHz to 15 MHz, which is suitable for removing dental plaque.

Furthermore, the amplitude of the AC signal Sac may be set to 0.1 mv to 3 V This is because if the amplitude of the AC signal Sac is less than 0.1 mV, it is difficult to expect the plaque removal effect, and if the amplitude of the AC signal Sac exceeds 3 V, toxins may be generated due to electrolysis of body fluids.

The filter 43 may perform a filtering operation on the AC signal Sac generated by the signal generator 42. For example, the filter 43 may include a low pass filter to convert the AC signal Sac in the form of a sawtooth wave to an AC signal Sac' in the form of a sine wave. However, the type of filter 43 is not limited to this, and various types of filters may be employed depending on the design structure.

The calibration part 44 may generate the driving signal Vd by mixing the DC signal Sdc with the AC signal Sac' supplied through the filter 43. For example, the calibration part 44 may be implemented as an operating amplifier capable of summing (or superimposing) the AC signal Sac' and the DC signal Sdc, but is not limited thereto.

Accordingly, the AC signal Sac' has an offset corresponding to the DC signal Sdc, and a driving signal Vd may be generated that has both an AC component and a DC component.

Since the driving signal Vd includes all the characteristics of the AC signal Sac, the driving signal Vd may be set at a frequency of 1 KHz to 1,000 MHz, and may also be set at a frequency of 5 MHz to 15 MHz, which is more suitable for removal of dental plaque. Furthermore, the amplitude of the driving signal Vd may be set to 0.1 mv to 3 V

Referring to FIG. 4A, the calibration part 440 may receive an AC signal Sac' having an amplitude of A volts V from the filter 43, and may superimpose a DC signal Sdc of B volts V as shown in FIG. 4B on that AC signal Sac' to generate a final driving signal Vd as shown in FIG. 4C.

In this case, the voltage value of the DC signal Sdc may be set above the amplitude of the AC signal Sac'. Accordingly, the voltage value of the driving signal Vd may be set above zero.

As a result, the DC offset value of the driving signal Vd may be set equal to or greater than the amplitude of the driving signal Vd.

On the other hand, the DC signal Sdc may be generated by the voltage divider 45. For example, the voltage distribution part 45 may receive the output voltage Vo of the DC-DC converter 41, and may perform voltage distribution on the output voltage Vo to generate the DC signal Sdc.

The voltage distribution part 45 may comprise, but is not limited to, a string of resistors for distributing the output voltage Vo.

If the output voltage Vo of the DC-DC converter 41 is suitable to be used directly for generating the driving signal Vd, the output voltage Vo may serve as the DC signal Sdc. In this case, the voltage divider 45 may be omitted, and the output voltage Vo of the DC-DC converter 41 may be input to the calibration part 44.

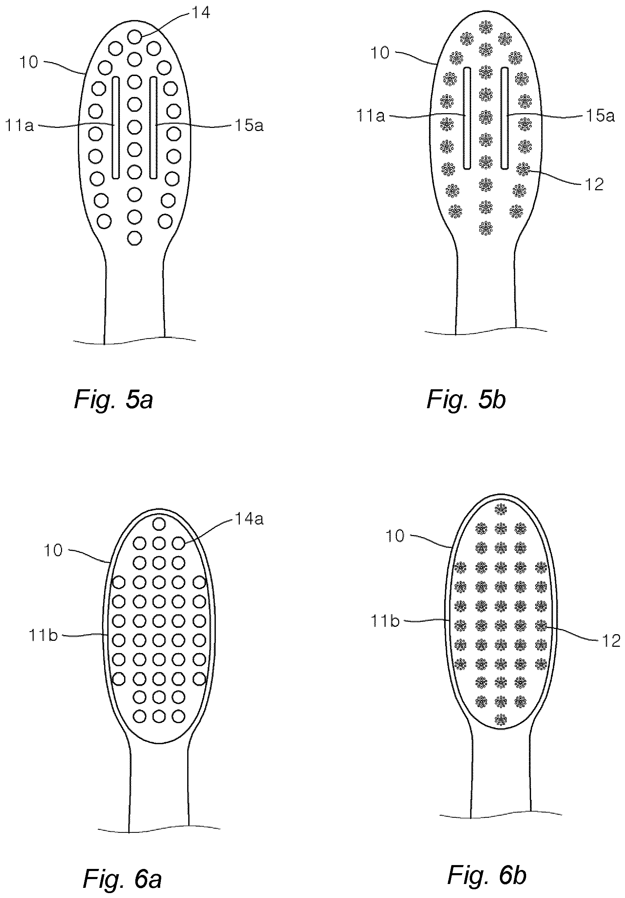

FIGS. 5A and 5B are views depicting a toothbrush head according to embodiments of the present invention. In particular, FIG. 5A is a view of a toothbrush head 10 without the conductive bristles 12 installed, and FIG. 5B is a view of a toothbrush head 10 with the conductive bristles 12 installed.

Referring to FIGS. 5A and 5B, an electrode 11a and an additional electrode 15a may be disposed on one side of the toothbrush head 10 according to embodiments of the present invention.

Furthermore, a plurality of bristle holes 14 may be formed around electrode 11a and additional electrode 15a, and a plurality of conductive bristles 12 may be disposed in each of these bristle holes 14.

Accordingly, the conductive bristles 12 may be spaced apart from the electrodes 11a and the additional electrodes 15a. However, when the user uses the electric toothbrush 1, the electrode 11a and the conductive bristles 12 are electrically connected via the user's saliva, toothpaste, or the like in the oral cavity, so that the driving signal Vd supplied to the electrode 11a can be transmitted to the conductive bristles 12.

The additional electrode 15a may be supplied with a driving signal Vd from the signal supply 40, or may be set to a separate ground potential.

These additional electrodes 15a, like the electrodes 11a, may be electrically connected with some of the conductive bristles 12 when the electric toothbrush 1 is in use.

FIGS. 6A and 6B are views depicting a toothbrush head according to other embodiments of the present invention. In particular, FIG. 6A is a view of a toothbrush head 10 without the conductive bristles 12 installed, and FIG. 6B is a view of a toothbrush head 10 with the conductive bristles 12 installed.

Referring to FIGS. 6A and 6B, an electrode 11B may be disposed on one side of the toothbrush head 10 according to embodiments of the present invention, and a plurality of bristle holes 14A may be formed on said electrode 11B.

A plurality of conductive bristles 12 may each be disposed in the bristle holes 14a of the electrode 11b, whereby the conductive bristles 12 are electrically connected to the electrode 11b through contact with the electrode 11b, so that the driving signal Vd supplied to the electrode 11b may be transmitted to the conductive bristles 12.

As shown in FIG. 6A, all of the bristle holes 14a may be formed on the electrode 11b. However, it is not limited to this, and instead, some of the bristle holes 14a may be formed on the electrode 11b and some of the remaining bristle holes (not shown) may be formed on one side of the toothbrush head 10.

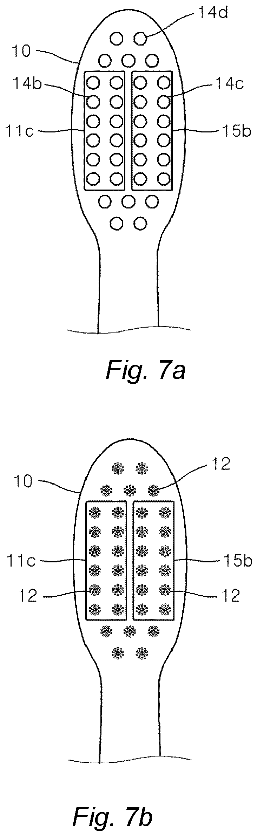

FIGS. 7A and 7B are views depicting a toothbrush head according to another embodiment of the present invention. In particular, FIG. 7A is a view of a toothbrush head 10 without the conductive bristles 12 installed, and FIG. 7B is a view of a toothbrush head 10 with the conductive bristles 12 installed.

Referring to FIGS. 7A and 7B, an electrode 11c and an additional electrode 15b may be disposed on one side of the toothbrush head 10 according to an embodiment of the present invention.

A plurality of first bristle holes 14b may be formed in electrode 11c, and a plurality of second bristle holes 14c may be formed in additional electrode 15b.

In each of these first bristle holes 14b and second bristle holes 14c, a plurality of conductive bristles 12 may be disposed.

Accordingly, the conductive bristles 12 disposed in the first bristle hole 14b are electrically connected to the electrode 11c through contact with the electrode 11c, so that the driving signal Vd supplied to the electrode 11c can be transmitted to the conductive bristles 12.

The additional electrode 15b may be supplied with a driving signal Vd from the signal supply 40, or may be set to a separate ground potential.

The conductive bristles 12 disposed in the second bristle hole 14c are electrically connected to the additional electrode 15b through contact with the additional electrode 15b, and thus can receive a driving signal Vd or ground voltage from the additional electrode 15b.

Meanwhile, a plurality of third bristle holes 14d may be further formed around electrode 11c and additional electrode 15b.

Conductive bristles 12 may be disposed in these third bristle holes 14d. However, by no means limited thereto, non-conductive bristles (not shown) may be disposed in the third bristle holes 14d formed in the toothbrush head 10.

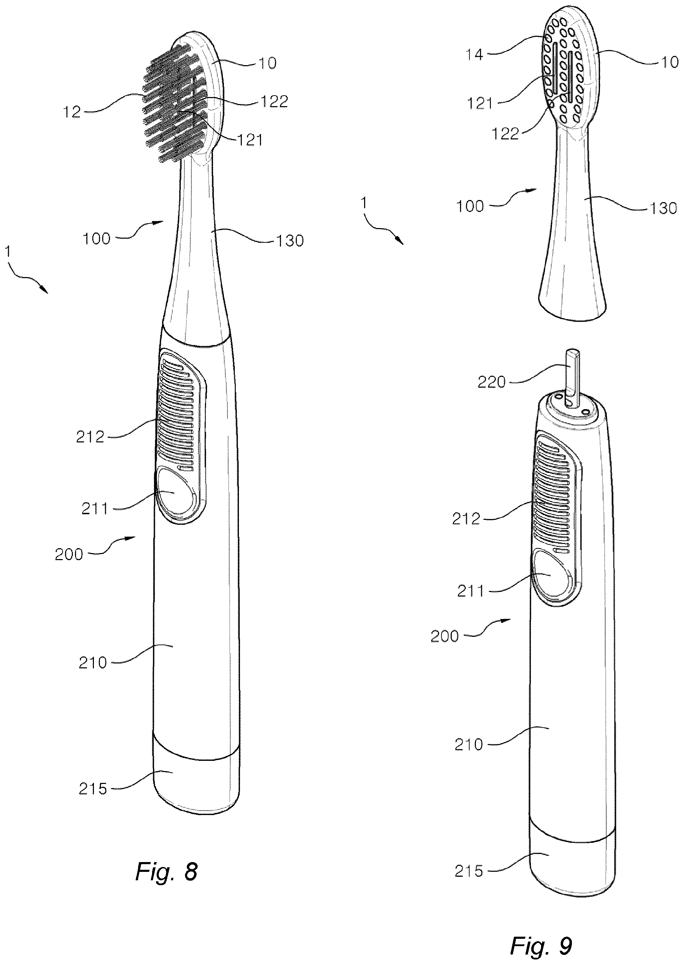

FIG. 8 is a view showing an electric toothbrush according to an embodiment of the present invention in a concrete form, and FIG. 9 is a view showing the electric toothbrush shown in FIG. 8 in a disassembled state. In particular, an electric toothbrush 1 having a toothbrush head 10 in the form shown in FIGS. 5A and 5B will be described hereinafter. However, the shape of the toothbrush head 10 may be changed to a shape such as that shown in FIGS. 6A and 6B, and FIGS. 7A and 7B.

Referring to FIGS. 8 and 9, an electric toothbrush 1 according to an embodiment of the present invention may include a head part 100 and a handle part 200.

The head part 100 may comprise a toothbrush head 10 and a head body 130, and may be designed to be engageable and detachable from the handle part 200.

Accordingly, if the head part 100 needs to be replaced due to aging or otherwise, the user can simply replace the existing head part 100 with a new head part 100.

The toothbrush head 10 may be disposed with conductive bristles 12, a first electrode 121, and a second electrode 122. Here, the first electrode 121 and the second electrode 122 may correspond to the previously described electrode 11a and the additional electrode 15a, respectively.

The conductive bristles 12 may be inserted and secured into a plurality of bristle holes 14 formed on the surface of the toothbrush head 10. The arrangement structure, number, size, etc. of these conductive bristles 12 is not particularly limited and can be varied in various forms.

The first electrode 121 and the second electrode 122 are spaced apart from each other and disposed on the surface of the toothbrush head 10, and may be disposed on the same plane as the conductive bristles 12.

A head body 130 may extend from the toothbrush head 10 to form the body of the head part 100. The head body 130 may be designed to a length suitable for use, and an end part of the head body 130 may be coupled to the handle part 200.

The handle part 200 is the main body of the electric toothbrush 1, which may be designed in such a way that it can be gripped by a user when in use.

Additionally, the handle part 200 may be coupled with the head part 100 and may have a separate retaining pin 220 for fixed engagement with the head part 100.

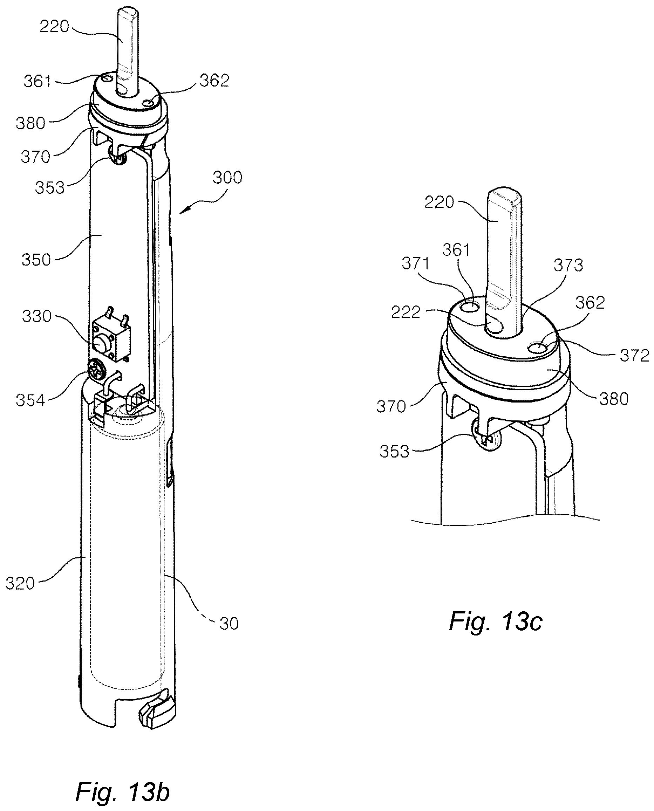

The interior of the handle part 200 may house a battery 30 (see FIG. 13B) for power supply, and the handle part 200 may be provided with a separate battery cap 215 for replacing the battery 30, for example.

Further, the handle part 200 may have an outer casing 210 for receiving and protecting the internal components, wherein a switch pressing area 211 and a non-slip part 212 may be formed in the outer casing 210.

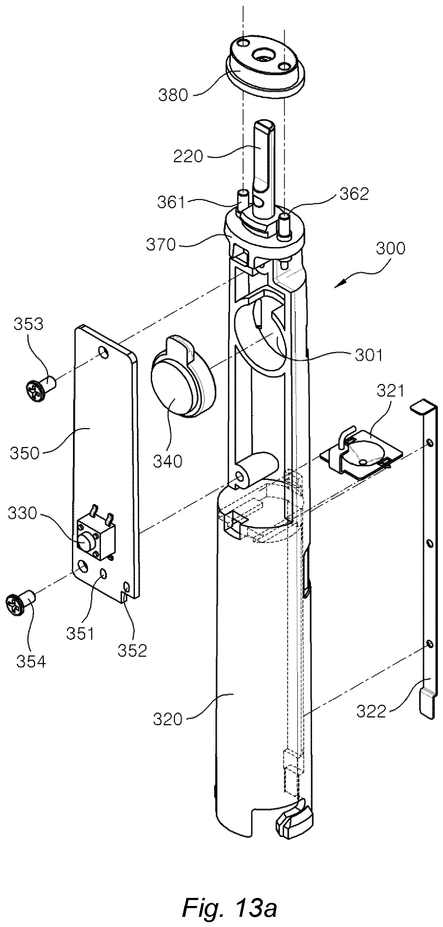

The switch pressing area 211 is formed in a position corresponding to an internally disposed switch 330 (see FIG. 13A), and a user can press the switch pressing area 211 to provide on/off control of the internal switch 330.

A user may turn on the power of the electric toothbrush 1 by pressing the switch pressing area 211 when brushing their teeth, whereby a driving signal Vd generated by the handle part 200 may be supplied to the first electrode 121 and/or the second electrode 122 of the head part 100 to generate an electric field for removing dental plaque.

Additionally, a driving signal Vd may be transmitted to the conductive bristles 12 via the first electrode 121 and/or the second electrode 122.

The non-slip part 212 is intended to provide a stable grip for the user, and may be designed with a structure and material to increase friction. For example, the non-slip part 212 may comprise a plurality of grooves.

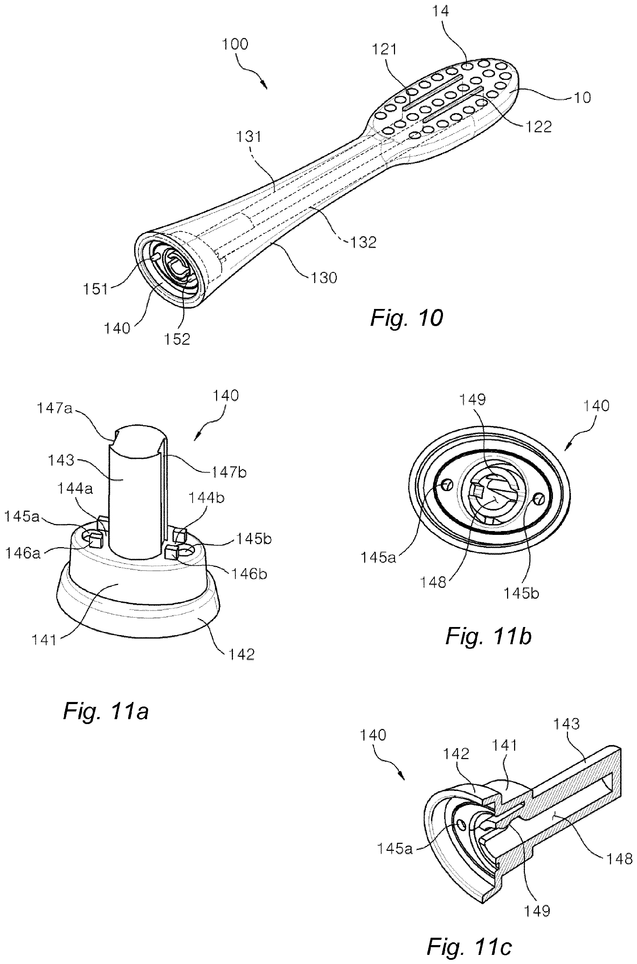

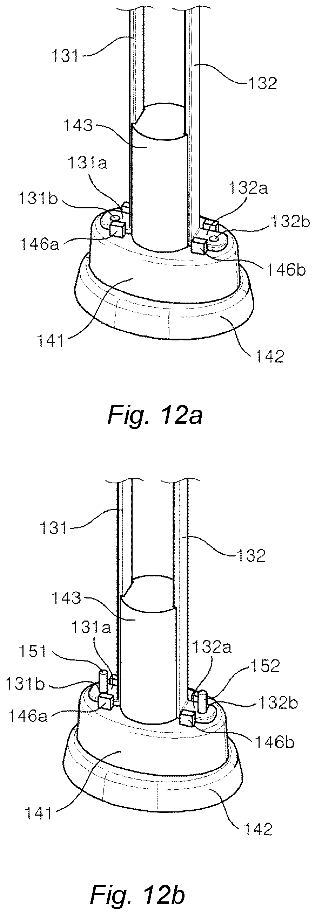

FIG. 10 is a view illustrating a head part according to an embodiment of the present invention, FIGS. 11A through 11C are views illustrating a head cover according to an embodiment of the present invention, and FIGS. 12A and 12B are views illustrating an engagement relationship of a head cover, a connection line, and a connection pin according to an embodiment of the present invention.

In particular, FIG. 11A illustrates head cover 140 from a top view, FIG. 11B illustrates head cover 140 from a bottom view, and FIG. 11C illustrates a cross-section of head cover 140.

Referring to FIG. 10, the head part 100 according to an embodiment of the present invention may include a first electrode 121, a second electrode 122, a toothbrush head 10, a head body 130, a first connection line 131, a second connection line 132, a head cover 140, a first connection pin 151, and a second connection pin 152.

The first electrode 121 and the second electrode 122 may be disposed on the toothbrush head 10, and in one example, the first electrode 121 and the second electrode 122 may be set as positive and negative electrodes, respectively.

The first electrode 121 and the second electrode 122 may have some projection from the toothbrush head 10, but are not limited thereto, as previously described.

The head body 130 may be integrally formed with the toothbrush head 10, and may be formed via Insert Injection Molding, for example, like the toothbrush head 10.

Inside the head body 130, a first connection line 131 and a second connection line 132 for transmitting the driving signal Vd may be disposed.

The first connection line 131 and the second connection line 132 may be connected with the first electrode 121 and the second electrode 122, respectively, in the interior of the toothbrush head 10. Furthermore, the first connection line 131 and the second connection line 132 may be formed to extend to an end side of the head body 130 to receive power from the handle part 200, and may be electrically connected with the first connection pin 151 and the second connection pin 152 disposed on the head cover 140.

For manufacturing convenience, the first connection wire 131 and the first electrode 121 may be integrally formed, and the second connection wire 132 and the second electrode 122 may also be integrally formed.

Further, the first connection line 131 and the second connection line 132 may be formed of the same material as the electrode material described above.

A head cover 140 may be coupled to an end part of the head body 130 to close the end part of the head body 130.

The first connection pin 151 and the second connection pin 152 are connected to the first connection line 131 and the second connection line 132, respectively, and may be partially exposed to the outside through the head cover 140.

When the head part 100 and handle part 200 are subsequently coupled, the connecting pins 151, 152 may contact the connection terminals 361, 362 (see FIG. 13A) disposed on the handle part 200.

Referring to FIGS. 11A through 11C, the head cover 140 may include a column part 143 having a retaining pin receiving groove 148 formed therein.

A retaining pin receiving groove 148 is formed on the lower surface of the head cover 140, which may later be engaged with the retaining pin 220 of the handle part 200.

In this case, a convex part 149 may be formed on the interior of the retaining pin receiving groove 148, such that when the retaining pin 220 is inserted into the interior of the retaining pin receiving groove 148, the convex part 149 may interlock with the concave part 222 of the retaining pin 220 (see FIG. 13C) to further enhance the engagement with the retaining pin 220.

Further, a column part 143 may be disposed at a center part of the first base part 141 and extend upwardly therefrom. On either side of such column part 143, a first resting region 144a and a second resting region 144b may be located, respectively.

Accordingly, a first resting region 144a and a second resting region 144b may be formed on the top surface of the first base part 141.

Additionally, at least one or more protrusions 146a, 146b may be disposed around the periphery of the first resting region 144a and the second resting region 144b, respectively.

For example, at least one first protrusion 146a surrounding a first resting region 144a and at least one second protrusion 146b surrounding a second resting region 144b may be formed on the top surface of the first base part 141.

In FIG. 11A, two first protrusions 146a and two second protrusions 146b are shown, but are not limited thereto, and the number of first protrusions 146a and second protrusions 146b can be varied.

This configuration of protrusions 146a, 146b allows the end parts 131a, 132a of each connecting wire 131, 132 to rest securely in their respective resting areas 144a, 144b.

A first pinhole 145a may be formed in the first resting region 144a, and a second pinhole 145b may be formed in the second resting region 144b.

Thus, the first connecting pin 151 can pass through the head cover 140 through the first pinhole 145a, and the second connecting pin 152 can pass through the head cover 140 through the second pinhole 145b.

Meanwhile, in the column part 143, a first guiding groove 147a and a second guiding groove 147b for guiding the first connection line 131 and the second connection line 132, respectively, may be formed along the longitudinal direction.

The second base part 142 is formed on the lower side of the first base part 141 and may be formed with a larger diameter than the first base part 141. However, the second base part 142 may be omitted if desired.

Referring to FIG. 12A, portions of the first connection line 131 and the second connection line 132 may be inserted into the first guide groove 147a and the second guide groove 147b of the column part 143, respectively, and the end part 131a of the first connection line 131 and the end part 132a of the second connection line 132 may be bent and rest in the first resting region 144a and the second resting region 144b, respectively.

In this case, the end part 131a of the first connection line 131 and the end part 132a of the second connection line 132 may be bent in opposite directions to each other, and may be provided with first connection holes 131b and second connection holes 132b corresponding to the first pinhole 145a and the second pinhole 145b, respectively.

For example, the end part 131a of the first connection line 131 may be rested in the first resting region 144a while bent in the first direction, such that the first connecting hole 131b formed in said end part 131a may be located over the first pinhole 145a in the first resting region 144a.

Furthermore, the end part 132a of the second connection line 132 is rested in the second resting region 144b while bent in a second direction opposite to the first direction, such that the second connecting hole 132b formed in said end part 132a can be located over the second pinhole 145b in the second resting region 144b.

Referring to FIG. 12B, a first connection pin 151 may be disposed through a first connection hole 131b of the first connection line 131 and a first pinhole 145a of the head cover 140, and a second connection pin 152 may be disposed through a second connection hole 132b of the second connection line 132 and a second pinhole 145b of the head cover 140.

One end of the first connecting pin 151 may be electrically connected to the end of the first connecting wire 131a via a soldering process near the first connecting hole 131b, and the other end of the first connecting pin 151 may protrude downwardly through the first pinhole 145a of the head cover 140.

One end of the second connection pin 152 may be electrically connected to the end 132a of the second connection wire 132 via a soldering process near the second connection hole 132b, and the other end of the second connection pin 152 may protrude downwardly through the second pinhole 145b of the head cover 140.

For example, the first connecting pin 151 and the second connecting pin 152 may be implemented as a pogo pin with an embedded spring.

After assembly as shown in FIG. 12B is complete, an insert injection molding process can be performed to form the toothbrush head 10 and head body 130.

FIG. 13A is an exploded view illustrating an internal configuration of a handle according to an embodiment of the present invention, FIG. 13B is a view illustrating the components shown in FIG. 13A combined, and FIG. 13C is a view illustrating a retaining pin and a connection terminal according to an embodiment of the present invention.

Referring to FIGS. 13A through 13C, the handle part 200 according to an embodiment of the present invention may include a retaining pin 220, an inner case 300, a battery 30, a switch 330, a vibration motor 340, a circuit board 350, a first connection terminal 361, a second connection terminal 362, and a top cover 380.

The inner case 300 is housed inside the outer case 210 and may provide areas and spaces for components such as the battery 30, switch 330, vibration motor 340, and circuit board 350 to be installed.

In the lower part of the inner case 300, a battery receiving part 320 in which the battery 30 is located is located, and a vibration motor mounting part 301 in which a vibration motor 340 is mounted on the upper side of said battery receiving part 320 may be provided. In addition, a plate-shaped coupling part 370 may be formed on the upper side of the inner case 300, on which the connection terminals 361, 362 and the retaining pin 220 are installed.

A first battery terminal 321 and a second battery terminal 322 may be installed in the battery receiving part 320, and the first battery terminal 321 and the second battery terminal 322 may be electrically connected with the circuit board 350, respectively. For example, the first battery terminal 321 may be connected to a first substrate terminal 351 of the circuit board 350, and the second battery terminal 322 may be connected to a second substrate terminal 352 of the circuit board 350.

The battery 30 may be inserted into the interior of the battery receiving part 320 and electrically connected to said battery terminals 321, 322.

Switch 330 is configured to control the power supply by battery 30 and may be installed on circuit board 350.

A vibration motor 340 may be disposed in the vibration motor seating portion 301 provided in the inner case 300, and may provide vibrations to alert a user to the operation of the electric toothbrush 1.

For example, when the switch 330 is turned on by a user, the vibration motor 340 may begin to operate and may continuously provide vibration until the switch 330 is turned off. However, the manner in which the vibration motor 340 operates is not limited to this, may be set in various ways.

Circuit board 350 may be fixedly installed in inner case 300 via fastening members 353, 354 and the like, and may be disposed between switch 330 and vibration motor 340. Accordingly, the switch 330 may be disposed on a top surface of the circuit board 350 and the vibration motor 340 may be disposed on a bottom surface of the circuit board 350.

In addition, the circuit board 350 may be mounted with a signal supply 40 that utilizes the voltage from the battery 30 to generate a driving signal Vd.

The signal supply 40 can generate the driving signal Vd by utilizing the voltage of the battery 30 supplied as the switch 330 is turned on. In addition, the signal supply 40 can provide the vibration motor 340 with the voltage required for operation of the vibration motor 340.

The first connection terminal 361 and the second connection terminal 362 are each disposed in a form that passes through the coupling part 370, and when the head part 100 and the handle part 200 are coupled, they can play a role in transmitting the driving signal Vd generated by the signal supply 40 to the head part 100 by contacting the first connection pin 151 and the second connection pin 152 of the head part 100, respectively.

To this end, the lower end of the downwardly projecting connection terminals 361, 362 of the coupling part 370 may be connected to the circuit board 350 on which the signal supply 40 is mounted, and the upper end of the upwardly projecting connection terminals 361, 362 of the coupling part 370 may be exposed to the outside through the top cover 380.

A retaining pin 220 may be disposed in the center of the coupling part 370 and formed to extend upwardly, and may be insertively coupled to the retaining pin receiving groove 148 provided in the head part 100. As previously described, the retaining pin 220 may be formed with a concave part 222 corresponding to the convex part 149 of the retaining pin receiving groove 148.

The top cover 380 may be installed over the coupling part 370 of the inner case 300 and may be formed of an insulating material, such as silicone, for example.

The top cover 380 may have a first opening 371 for exposing the first connection terminal 361 to the outside, a second opening 372 for exposing the second connection terminal 362 to the outside, and a third opening 373 through which the retaining pin 220 may pass.

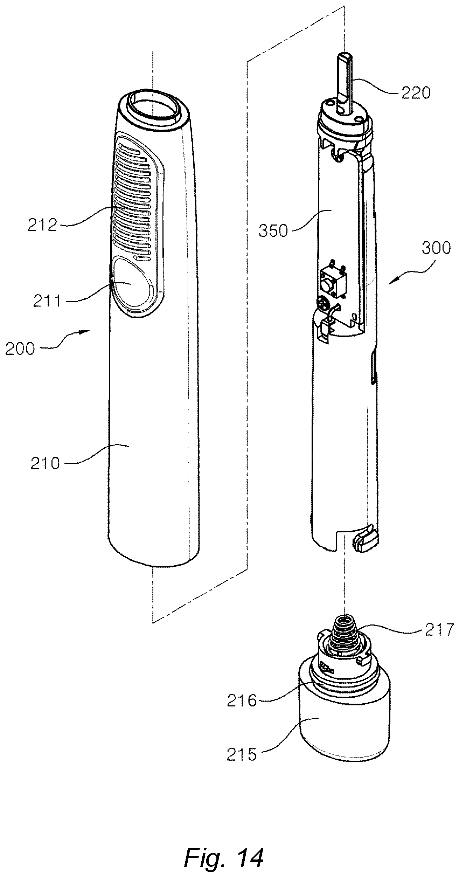

FIG. 14 is a view illustrating the coupling relationship of a battery cap, an inner case, and an outer case in accordance with embodiments of the present invention.

Referring to FIG. 14, the assembled inner case 300, including the switch 330, vibration motor 340, circuit board 350, and the like, may have an outer case 210 installed outwardly therefrom, and a battery cap 215 may be coupled to the lower portion of the inner and outer cases 300, 210.

The battery cap 215 may have an O-ring 216 for sealing and a spring 217 electrically connected to the second battery terminal 322.

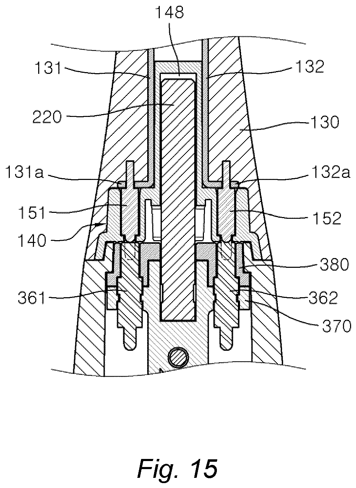

FIG. 15 is a partial cross-sectional view of an electric toothbrush when the head and handle parts are combined, according to an embodiment of the present invention.

Referring to FIG. 15, when the head part 100 and the handle part 200 are coupled, the retaining pin 220 is insertively coupled into the retaining pin receiving groove 148. Further, the first connecting pin 151 associated with the end part 131a of the first connection line 131 may be contacted at the first connection terminal 361, and the second connecting pin 152 associated with the end part 132a of the second connection line 132 may be contacted at the second connection terminal 362.

Thereafter, the internal switch 330 is turned on by the user pressing the switch pressing area 211, and the signal supply 40 may generate the driving signal Vd and supply it to the first connection line 131 and the second connection line 132 via the circuit board 350 and the connection terminals 361, 362. Accordingly, the first electrode 121 and the second electrode 122 may form an electric field for removing the dental plaque based on the supplied driving signal Vd.

Those skilled in the art to which the present invention pertains will understand that the present disclosure can be embodied in other specific forms without changing its subject matter or essential features. Therefore, it should be understood that the embodiments described above are illustrative only and not restrictive. The scope of the present invention is defined by the claims below rather than the detailed description above, and all changes or modifications derived from the claims and their equivalents should be construed as being included in the scope of the present invention.

## Claims

1. An electric toothbrush comprising:
a toothbrush head having a plurality of conductive bristles and at least one electrode disposed thereon that is supplied with a driving signal;
a handle part connected to the toothbrush head and having a user-grippable shape;
a battery disposed inside the handle part; and
a signal supply located inside the handle part and configured to receive a voltage from the battery to generate an AC signal and a DC signal, and to generate the driving signal comprising a mixture of the AC signal and the DC signal to supply the electrodes.

2. The electric toothbrush according to claim 1, wherein the conductive bristles comprise a synthetic resin and a conductive material.

3. The electric toothbrush according to claim 2, wherein the conductive material comprises graphene.

4. The electric toothbrush according to claim 1, wherein the conductive bristles are spaced apart from the electrodes.

5. The electric toothbrush according to claim 4, wherein , in use of the electric toothbrush, the electrodes and the conductive bristles are electrically connected through saliva and toothpaste in the oral cavity so that the driving signal from the electrodes is transmitted to the conductive bristles.

6. The electric toothbrush according to claim 1, wherein a plurality of first bristle holes are formed, and
at least a portion of the conductive bristles are electrically connected to the electrodes by insertion into the first bristle hole.

7. The electric toothbrush according to claim 6 further comprising:
an additional electrode spaced apart from the electrode and disposed on the toothbrush head,
wherein the additional electrodes are formed with a plurality of second bristle holes into which at least another portion of the conductive bristles are inserted.

8. The electric toothbrush according to claim 7, wherein the additional electrode is supplied with the driving signal or set to a ground potential.

9. The electric toothbrush according to claim 1, wherein the signal supply includes:
a DC-DC converter configured to take the voltage from the battery and convert it to an output voltage;
a signal generator configured to generate the AC signal using the output voltage of the DC-DC converter;
a filter configured to perform a filtering operation on the AC signal generated by the signal generator; and
a calibration part configured to mix the DC signal with an AC signal supplied through the filter to generate the driving signal.

10. The electric toothbrush according to claim 9, wherein the signal supply further comprises:
a voltage divider configured to distribute an output voltage of the DC-DC converter to generate the DC signal.

11. The electric toothbrush according to claim 9, wherein a frequency of the driving signal is between 1 KHz and 1,000 MHz.

12. The electric toothbrush according to claim 11, wherein an amplitude of the driving signal is between 0.1 mv and 3 volts.

13. The electric toothbrush according to claim 12, wherein a voltage value of the DC signal is set above an amplitude of the AC signal.
